(19) Europäisches Patentamt
European Patent Office
Office européen des brevets

(11) **EP 3 287 083 B1**

(12) **EUROPEAN PATENT SPECIFICATION**

(45) Date of publication and mention
of the grant of the patent:
**07.08.2019   Bulletin 2019/32**

(51) Int Cl.:
***A61B 17/16*** *(2006.01)*

(21) Application number: **16185505.1**

(22) Date of filing: **24.08.2016**

(54) **PERFORATOR ASSEMBLY**

LOCHERANORDNUNG

ENSEMBLE DE PERFORATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**28.02.2018   Bulletin 2018/09**

(73) Proprietor: **EMD Kft.**
**4031 Debrecen (HU)**

(72) Inventor: **Ujvári, Mihály Gyula**
**4032 Debrecen (HU)**

(74) Representative: **Kacsuk, Zsófia**
**Kacsukpatent Kft.**
**Üteg u. 11/a**
**1139 Budapest (HU)**

(56) References cited:
**WO-A1-2015/150844**

## Description

The present invention relates to a perforator assembly for perforating bone tissue, which perforator assembly comprises a drive shaft having a rotation axis, a drill head which is coaxial with the rotation axis, and a chipping head arranged coaxially around the drill head.

The perforator assembly is a medical device which can be used to perform trepanation on the crane i.e. drill a hole therein, and in particular to perform craniotomy after the trepanation. During the trepanation of the crane it is a problem, that substantial pressure has to be applied for advancing the drill head, however, the advancing of the drill head needs to be stopped immediately after perforation of the bone in order to prevent the drill head from penetrating and damaging the dura mater. In order to carry out surgical closure successfully it is a prerequisite that the dura mater must stay undamaged to be able to create fluid (liquor) sealing seam thereon.

A further problem is that the bone tissue of the crane is not a homogenous structure. The crane typically comprises a more dense outer and inner bone plate and a looser, porous inner soft bone layer therebetween, the so-called spongiosa. When designing the structure of the perforator assembly it must be ensured that the quality and performance of the drilling stays the same within the different tissue structures, while at the same time the drilling process must be interrupted as soon as the inner bone plate is penetrated (drilled through) which is rather thin as compared to the other tissue structures lying thereover, in order to protect the dura tissue right underneath of the inner bone structure as explained above. The cutting edge should be designed such that when the inner bone plate is penetrated a disk like formation remains, which further protects the dura tissue.

This problem is solved by the perforator assembly according to patent application WO2015/150844 in such a way that the drill head is surrounded by a coaxial chipping head and the two are connected by a bolt pin, which extends through the drill head and terminates in a helical groove formed in the wall of the chipping head. The drill head is connected to a drive shaft via cooperating connecting profiles provided on the distal end of the drive shaft and the proximal end of the drill head. Furthermore a spring is arranged in a nest formed between the distal end of the drive shaft and the proximal end of the drill head, which spring is compressed when the connecting profiles are abutting each other. If the drill head is pressed against the cranial bone, as a result of the counter pressure, the spring is compressed, the bolt pin is displaced in the proximal direction, and the connecting profiles abut each other, whereby the drive shaft rotates both the drill head and the chipping head via the bolt pin. The drill head extends beyond the chipping head, whereby the drill head is the first to penetrate through the cranial bone. When this happens, the counter force acting on the drill head is reduced drastically, thus the rotational speed of the drill head increases with respect to the chipping head. Consequently, the end of the bolt pin advances such that it is guided by the proximal edge of the helical groove in the chipping head allowing the drill head to advance in the distal direction. This displacement is further facilitated by the biased spring. As a result of the distal displacement the connecting profiles separate from each other and become disconnected, whereby the driving torque acting on the drill head and the chipping head ceases. In absence of any driving torque the bone tissue stops the chipping head and the drill head.

The biasing spring helped to disengage the drill head from the drive shaft, however, it rendered the cutting motion less balanced; moreover, it was very difficult to dimension the perforator assembly so as to cut through the inner bone plate of the bone tissue before the drilling is automatically stopped. To overcome this problem patent application WO2015/150844 proposes the use of a security spring, which counterbalances the effect of the biasing spring.

The inventor of the present invention has found that the incorporation of the security spring, although helpful in some respect, still does not ensure optimal working properties, and the additional structural element increases the complexity of the perforator assembly and renders it more difficult to disassemble and reassemble and to clean the pieces, in particular the springs and surrounding cavities.

The inventor of the present invention has realised that the disadvantageous working properties of the prior art perforator assemblies are due to the linear displacement dependence of the spring force, and it would be more desirable to provide a force profile with a steeper decay rate.

It is an objective of the present invention to overcome the problems associated with the prior art.

This objective is achieved by a perforator assembly according to claim 1.

It is a further objective of the present invention to reduce the number of separate pieces making up the perforator assembly.

This objective is further achieved by a perforator assembly according to claim 12.

Preferred embodiments of the invention are defined in the attached dependent claims.

Further details of the invention will be explained by way of exemplary embodiments with reference to the figures.

Fig. 1a is a partially cut-away side view of a perforator assembly according to the invention.
Fig. 1b is an exploded view of the perforator assembly according to Fig. 1a.
Fig. 2a is a side view of the drive shaft of the perforator assembly according to Fig. 1a.
Fig. 2b is a perspective view of the drive shaft of the perforator assembly according to Fig. 1a.
Fig. 2c is a top plan view of the distal end of the drive shaft of the perforator assembly according to Fig. 1a.

Fig. 3a is a side view of the drill head of the perforator assembly according to Fig. 1a without the bolt pins.
Fig. 3b is a perspective view of the drill head according to Fig. 3a.
Fig. 4a is a side view of the chipping head of the perforator assembly according to Fig. 1a without a distal casing portion.
Fig. 4b is a perspective view of the chipping head according to Fig. 4a.
Fig. 5a is a partially cut-away side view of the perforator assembly according to Fig. 1a in a drill position.
Fig. 5b is a partially cut-away side view of the perforator assembly according to Fig. 1a in a disengaged position.

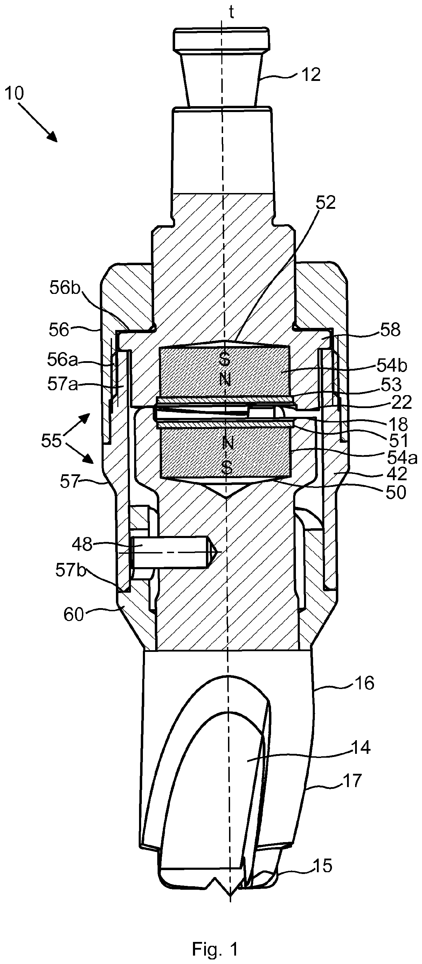

Fig. 1a depicts a preferred embodiment of a perforator assembly 10 according to the invention, which comprises a drive shaft 12 with rotation axis t, a drill head 14 having the same rotation axis t and a chipping head 16 arranged coaxially around the drill head 14. The proximal end of the drive shaft 12 may be connected to a revolution reducer, so-called TREAPAN adapter, coupled with a drive motor (not shown), and may be formed for example as a Hudson cone. The drill head 14 and the chipping head 16 each comprise a distal cutting edge 15 and 17 respectively, and the distal cutting edge 15 of the drill head 14 extends beyond the distal cutting edge 17 of the chipping head 16.

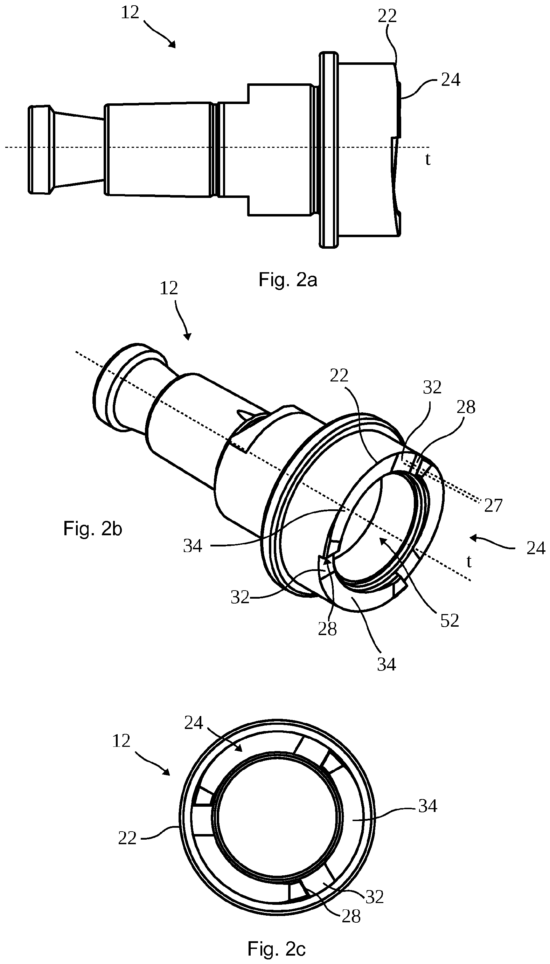

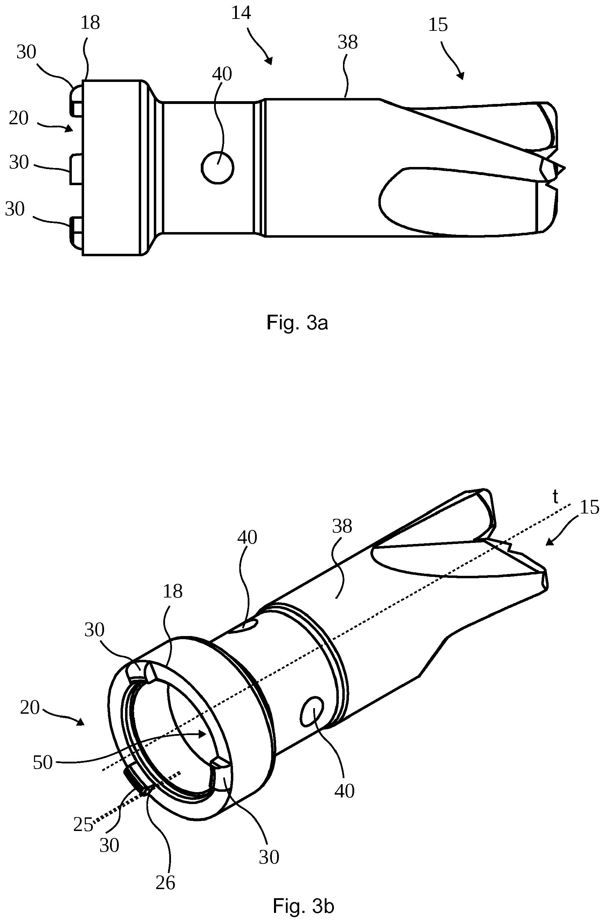

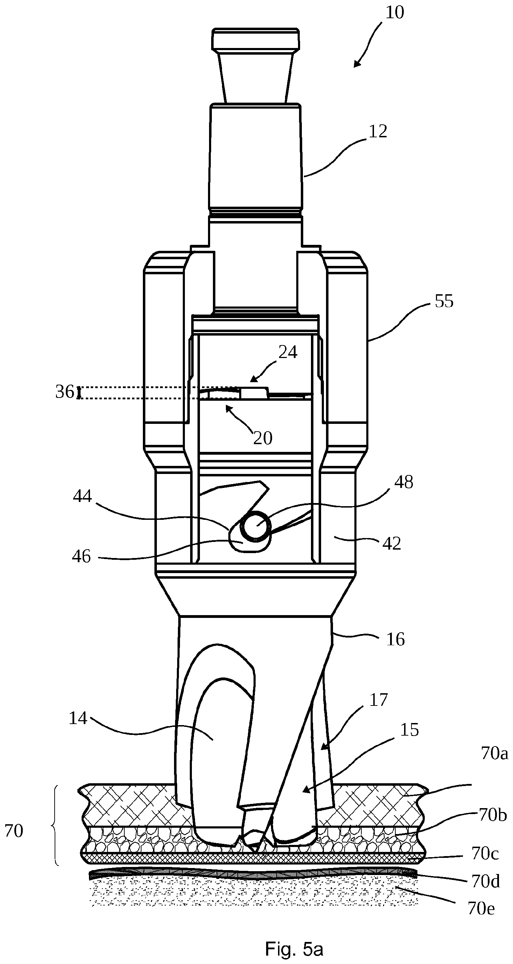

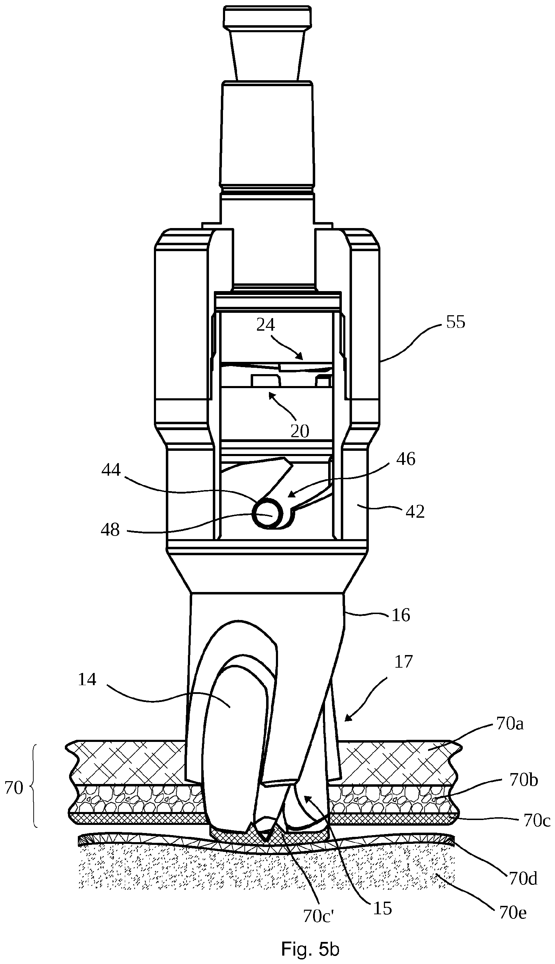

A first connecting profile 20 is provided on a proximal end 18 of the drill head 14 (see Figs. 3a, 3b), while a second connecting profile 24 is provided on a distal end 22 of the drive shaft 12 (see Figs. 2a, 2b). The drill head 14 is arranged so as to be displaceable along the rotation axis t with respect to the chipping head 16 and with respect to the drive shaft 12 at the same time between a proximal drill position (see Fig. 5a) and a distal disengaged position (see Fig. 5b). In the proximal position the second connecting profile 24 on the distal end 22 of the drive shaft 12 engages the first connecting profile 20 and cooperates therewith such as to transmit rotational motion from the drive shaft 12 to the drill head 14. In the distal position the first connecting profile 20 on the proximal end 18 of the drill head 14 and the second connecting profile 24 on the distal end 22 of the drive shaft 12 are disengaged, whereby the driving torque ceases as will be explained later on.

The first connecting profile 20 formed on the drill head 14 comprises at least one, preferably at least two, more preferably three projections 30 for coupling with corresponding recesses 32 of the second connecting profile 24. Each projection 30 is provided with an abutting surface 26 defined by generating lines 25 that are substantially parallel with the rotational axis t.

Each recess 32 of the second connecting profile 24 on the drive shaft 12 comprises a driving surface 28 defined by generating lines 27 that are preferably substantially parallel with the rotational axis t. Preferably, the same number of driving surfaces 28 are provided as abutting surfaces 26.

Only a few exemplary generating lines 26 and 27 are depicted in Figs. 2b and 3b respectively, and in the case of the present embodiment these define rectangular abutting surfaces 26 and driving surfaces 28, respectively. As will be explained in more detail later on the abutting surfaces 26 and the driving surfaces 28 need to satisfy basically two requirements: they must allow transmission of the rotational motion of the drive shaft 12 to the drill head 14 and they must allow relative displacement of the drive shaft 12 and the drill head 14 along the rotational axis t. Consequently, the abutting surface 26 and the drive surface 28 need not necessarily be planar surfaces, they can be for example curved surfaces complementing each other, for example wave-like surfaces. Continuousness of the surfaces is also not required, the surface may have interruptions, ribs, perforations, etc. Furthermore, the abutting surfaces 26 and the drive surfaces 28 need not lie in a radial direction in order to transmit rotational motion.

According to the present invention the three abutting surfaces 26 and the three driving surfaces 28 delimit three projections 30 and three 32 recesses respectively. The recesses 32 of the drive shaft 12 are formed with a wedge surface 34 on their side opposite the driving surface 28. The dimension of the recesses 32 are preferably such that each recess 32 is adapted for receiving the corresponding projections 30 of the first connecting profile 20 of the drill head 14. Naturally, an inverted design is also conceivable, wherein the first connecting profile 20 of drill head 14 comprises recesses and the second connecting profile 24 of the drive shaft 12 comprises projections.

Preferably, the projections 30 of the first connecting profile 20 of the drill head 14 and the recesses 32 of the second connecting profile 24 of the drive shaft 12 are formed rotation-symmetrically with respect to the rotational axis t (i.e. formed evenly spaced around the circumference of a circle), whereby the proximal end 18 of the drill head 14 may engage the distal end 22 of the drive shaft 12 in more than one position. Engagement is understood to refer to the situation wherein the projections 30 of the drill head 14 are received in the recesses 32 of the drive shaft 12 and the abutting surfaces 26 and the driving surfaces 28 abut each other.

In case of the present embodiment the three projections 30 are evenly spaced along the circumference of the proximal end 18, being in positions rotated by 120 degrees with respect to each other. In case a number of n projections 30 are provided those are preferably rotated by 360/n degrees with respect to the neighbouring projection 30. The connecting profile 24 of the distal end 22 of the drive shaft 12 is designed to correspond to the number and arrangement of the abutting surfaces 26 and the projections 30. Accordingly, the recesses 32 of the drive shaft 12 are preferably also formed rotation-symmetrically and are evenly spaced along the circumference of the distal end 22 of the drive shaft 12.

When the connecting profile 20 of the drill head 14 engages the connecting profile 24 of the drive shaft 12 the abutting surface 26 and the driving surface 28 abutting each other overlap in the direction of the rotational axis t and contact each other along a connection length 36 (see Fig. 5a). If the length of the abutting surface 26 and that of the driving surface 28 is the same along the rotational axis t then the overlap is complete and the connection length equals the height of the projection 30, otherwise the shorter element determines the connection length 36. The connection length 36 determines the distance by which the proximal end 18 of the drill head 14 must be displaced from the distal end 22 of the drive shaft 12 in order to terminate the torque transmission connection between the two. Preferably, the connection length 36 (i.e. the length of the abutting surface 26 or the driving surface 28, which ever is shorter, measured along the rotational axis t) is between 0.4 to 1 mm, preferably about 0.5 to 0.6 mm. The drill head 14 can be displaced along the rotational axis t by a distance corresponding to the connection length 36 before the drive mechanism is disengaged.

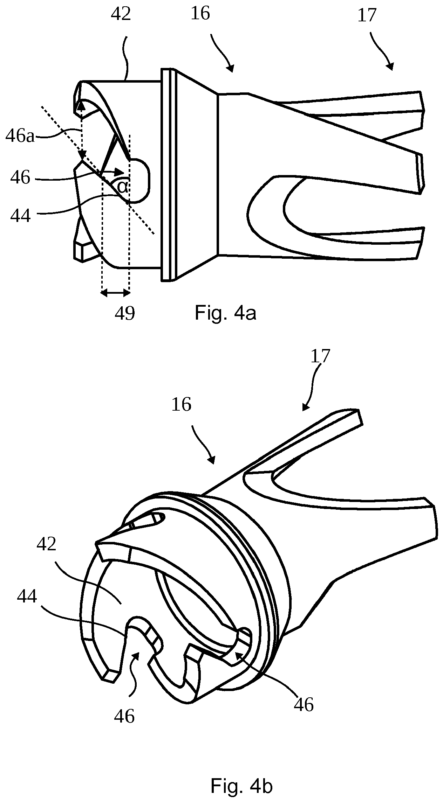

Disengagement of the drive mechanism can be ensured by providing a connection between the drill head 14 and the chipping head 16, which transforms relative rotation of the drill head 14 and the chipping head 16 around the rotational axis t into relative displacement along the rotational axis t. According to an exemplary embodiment this connection is ensured by a pin-groove cooperation. At least one groove 46 with a proximal helical edge 44 corresponding to the driving direction is provided in a cylindrical wall 42 of the chipping head 16. An angle α between the helical edge 44 and the rotational axis t is preferably 40 to 50 degrees. In case of the embodiment depicted in Figs. 4a and 4b this groove 46 is a helical groove formed through the wall 42, however, grooves 46 with other shapes may be applied as well, which have a suitable proximal helical edge 44, such as triangle shaped grooves 46.

The drill head 14 comprises at least one bore hole 40 extending through an external surface 38 of the drill head 14. The connection between the drill head 14 and the chipping head 16 is ensured by a bolt pin 48 arranged in the bore hole 40 of the drill head 14. When the drill head 14 is located inside the chipping head 16, the pin 48 extends into the groove 46 of the chipping head 16, whereby the bolt pin 48 is guided by the proximal helical edge 44. The groove 46 preferably opens in the direction of the proximal end of the chipping head 16, and the opening 46a has a greater diameter than the diameter of the bolt pin 48, whereby the drill head 14 can be inserted into the chipping head 16 after the bolt pin 48 is secured in the bore hole 40 of the drill head 14 as will be explained later on.

The helical edge 44 is formed such that its elevation 49 is greater than the connection length 36 (preferably by at least 0.3 to 0.5 mm) in order to provide for disengagement of the first connection profile 20 of the proximal end 18 of the drill head 14 from the second connection profile 24 of the of the distal end 22 of the drive shaft 12 such as to terminate the torque transmission connection between the two when the bolt pin 48 is guided to its distal position (see Fig. 5b) along the trajectory defined by the edge 44. The working length of the helical edge 44 (i.e. the portion along which the bolt pin 48 is guided from a first position corresponding to the proximal position of the drill head 14 and a second position corresponding to the distal position of the drill head 14) may be substantially shorter than its full length, e.g. the full length may include a portion which leads to the opening 46a but which is no longer accessible to the bolt pin 48 in the assembled position of the perforator assembly 10.

The perforator assembly 10 is further provided with a mechanism for biasing the proximal end 18 of the drill head 14 from the distal end 22 of the drive shaft 12 when the perforator assembly 10 is assembled. According to the present embodiment cavities 50 and 52 are provided in the proximal end 18 of the drill head 14 and in the distal end 22 of the drive shaft 12 respectively, and magnets 54a and 54b, with north poles N and south poles S are housed in the cavities 50, 52. The cavities 50 and 52 are preferably sealed by closing plates 51 and 53 respectively for retaining the magnets 54a and 54b. The closing plates 51 and 53 preferably provide a permanent and preferably hermetic sealing connection, whereby the drive shaft 12 and the drill head 14 can be cleaned without removing the closing plates 51 and 53 and the magnets 54a and 54b.

The magnets 54a and 54b are arranged such that the same magnetic poles (north poles N in Fig. 1a) are facing each other, whereby the magnets 54a, 54b repel each other when the proximal end 18 of the drill head 14 is pushed in the direction of the distal end 22 of the drive shaft 12 in order to engage the respective connecting profiles 20 and 24. The repelling force increases roughly inversely proportionally to the square of the distance r between the centres of the two magnets 54a, 54b:

$$F \sim \frac{1}{r^2}$$

The magnets 54a, 54b are preferably permanent magnets made of neodymium. According to a preferred embodiment the thickness of the magnets 54a, 54b is 0.3 - 0.7 mm, preferably approx. 0.4 mm, and the remanence of the magnets 54a, 54b is preferably between 1100 mT and 1300 mT, preferably about 1200 mT, for example the magnets 54a, 54b may be of the grade N35SH.

The drive shaft 12 and the chipping head 16 is preferably arranged at a constant distance with respect to each other which is preferably ensured with a casing 55 surrounding and connecting the drive shaft 12 and the chipping head 16 (see Fig. 1a. According to the present embodiment the casing 55 comprises a proximal portion 56 and a distal portion 57, which are provided with cooperating threads 56a and 57a forming a screw connection for connecting the two portions 56, 57. The proximal portion 56 is further provided with a proximal flange 56b that abuts a collar 58 of the drive shaft 12 as illustrated in Fig. 1a. The distal portion 57 is attached to the chipping head 16, preferably in a permanent way, for example in the present embodiment a distal end face 57b of the distal portion 57 is welded to a circumferential shoulder 60 of the chipping head 16. This has the advantage of reducing the number of pieces to be assembled and disassembled in case of a perforator assembly that can be used more than once.

In order to further reduce the number of the pieces the pin 48 is preferably pressed into the bore hole 40 of the drill head 14 and permanently fixed therein, for example by a suitable adhesive or by welding. This way the drill head 14 and the one or more pins 48 can be fitted into the chipping head 16 as a single piece. The drill head 14 is inserted into the tube-like chipping head 16 and rotated to a position where the pins 48 are each next to one of the openings 46a of the grooves 48 formed in the side wall 42 of the chipping head 16. In this position the drill head 14 can be advanced in the direction of the distal cutting edge 17 of the chipping head 16, while the pins 48 are guided by the grooves 46 to their distal position.

Once the drill head 14 has been inserted into the chipping head 16, the assembly can be secured to the drive shaft 12 via the casing 55. The proximal portion 56 of the casing 55 is pulled over the drive shaft 12 until its proximal flange 56b abuts the collar 58 of the drive shaft 12. The drive shaft 12 and the assembly formed by the drill head 14 and the chipping head 16 are then secured to each other by screwing the proximal casing portion 56 onto the distal casing portion 57 which is preferably permanently attached to the chipping head 16.

According to the present invention the biasing mechanism is provided in the form of magnets 54a, 54b arranged inside the cavities 50, 52 at the respective ends of the drill head 14 and the drive shaft 12 facing each other. The number of pieces to be assembled can be further reduced by sealing off the magnets 54a, 54b via the closing plates 51, 53 respectively, in a permanent manner, e.g. via an adhesive or by welding.

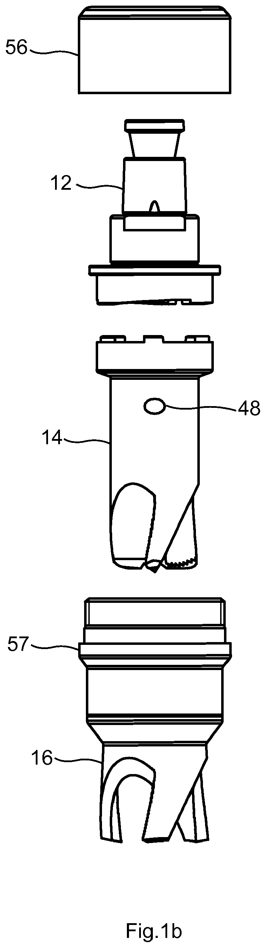

In this way a four-piece perforator assembly 10 can be obtained the pieces of which are illustrated in the exploded view of Fig. 1b. The four-piece perforator assembly 10 according to the invention comprises:

- a first piece being the drive shaft 12 with rotation axis t,
- a second piece being the chipping head 16 for arranging with the same rotation axis t next to the drive shaft 12 and being permanently provided with the distal casing portion 57 at its proximal end,
- a third piece being the drill head 14 for coaxially inserting into the chipping head 16 and being provided with the bolt pin 48 extending from the outer surface of the side wall of the drill head 14, and
- a fourth piece being the proximal casing portion 55 for holding the pieces together.

This means a substantial reduction of the number of separate pieces as compared to prior art perforator assemblies.

All the components of the perforator assembly 10 may be made of metal, which further simplifies the cleaning after use.

The use of the perforator assembly 10 according to the invention will now be described with reference to Figs. 5a and 5b.

The perforator assembly 10 is used for drilling bone tissue 70, in particular cranial bone tissue. The bone tissue 70 comprises the following successive layers starting from the external (proximal) side to the internal (distal) side: external bone plate 70a, spongiosa 70b (porous inner soft bone layer), internal bone plate 70c. This is followed by the dura tissue 70d, which should not be penetrated during drilling and which is bordering the inner brain water 70e.

Before starting the drilling the drive shaft 12 of the perforator assembly 10 is fixed in a revolution reducing adapter coupled with a drill motor, then the distal cutting edge 15 of the drill head 14 is pressed against the bone tissue 70. As a result, the drill head 14 is displaced in the proximal direction with respect to the chipping head 16 along the rotational axis t until the drill head 14 abuts th drive shaft 12 and reaches its proximal position. As the drill head 14 is forced in the proximal direction the wedge surface 34 on the second connecting profile 24 automatically directs the projections 30 of the first connection profile 20 into the recesses 35, i.e. into the position wherein the abutting surfaces 26 abut the driving surfaces 28. In this position the abutting surfaces 26 of the first connecting profile 20 on the proximal end 18 of the drill head 14 and the driving surfaces 28 of the second connecting profile 24 on the distal end 22 of the drive shaft 12 abut each other along the whole of the connection length 36 and the bolt pin 48 extending through the drill head 14 preferably also reaches its proximal working position (first position) illustrated in Fig. 5a. During this displacement the magnets 54a, 54b arranged within the cavities 50 and 52 approach each other, whereby the repelling force between the magnets 54a, 54b increases. In the proximal position of the drill head 14 the perforator assembly 10 is ready for drilling.

When starting the drill motor, the drive shaft 12 starts to rotate and the driving surfaces 28 of its second connecting profile 24 exert drive torque on the abutting surfaces 26 of the first connecting profile 20 on the proximal end 18 of the drill head 14, as a result of which the drill head 14 starts to rotate around the rotational axis t, and therewith the chipping head 16 as well, which is connected with the drill head 14 via the bolt pins 48. By continuously pushing the perforator assembly 10 in the distal direction into the bone tissue 70, first the distal cutting edge 15 of the rotating drill head 14, and then the distal cutting edge 17 of the co-rotating chipping head 16 start to cut into the bone tissue 70 and gradually advance therein.

In case of drilling a cranial bone, at the end of the drilling, when the distally extending cutting edge 15 of the drill head 14 has cut through the spongiosa 70b of the bone tissue 70 and starts to penetrate the internal bone plate 70c of the bone tissue 70, the counter force acting on the drill head 14 drastically decreases while the cutting edge 17 of the chipping head 16 is still inside the external bone structure 70a - or having drilled there through, it is still in the spongiosa 70b - requiring substantial driving torque to be rotated therein.

The drill head 14 exerts torque on the chipping head 16 by way of the bolt pins 48. Because the groove 46, which receives the bolt pins 48, has a helical proximal edge 44 in the direction of the rotation, hence the counter force acting on the bolt pins 48 comprises a force component which is parallel with the rotational axis t and is directed in the distal direction (i.e. in the direction of the bone tissue 70), which, in absence of a counter force exerted by the bone tissue 70, advances the drill head 14 in the distal direction (in the direction of the bone tissue 70) with respect to the chipping head 16 until the bolt pins 48 reach their distal end position corresponding to the distal position of the drill head 14 as can be seen in Fig. 5b. In other words, the relative rotation of the drill head 14 and the chipping head 16 around the rotational axis t is transformed into relative displacement along the rotational axis t with the help of the helical edge 44 of the groove 46, which edge 44 is formed in the direction of the rotation, and with the help of the bolt pins 48, which are displaceable in the groove 46.

Since the casing 55 holds the drive shaft 12 at a constant distance from the chipping head 16, thus the drill head 14 is not only displaced relative to the chipping head 16 but is also displaced relative to the drive shaft 12. Consequently, the abutting surfaces 26 of the first connecting profile 20 of the drill head 14 are gradually shifted along the driving surfaces 28 of the second connecting profile 24 of the drive shaft 12. As long as the extent of shifting is less then the connection length 36, the drill head 14 is still driven by the drive shaft 12, accordingly, by suitable dimensioning of the connection length 36 it is possible to achieve that the drill head 14 cuts partially or wholly through the internal bone plate 70c while shifting in the distal direction. When the extent of the shifting exceeds the connection length 36, the abutting surfaces 26 of the first connecting profile 20 of the drill head 14 disengage the driving surfaces 28 of the second connecting profile 24 of the drive shaft 12, whereby the driving torque ceases to act on the drill head 14, and together with it, on the chipping head 16. In absence of the driving torque the chipping head 16 is no longer able to rotate in the bone tissue. Thus the chipping head 16, and the drill head 14 connected therewith by the bolt pin 48, come to a halt, whereby the perforator assembly 10 only just cuts through the internal bone plate 70c of the bone tissue 70, and automatically stops thereafter, in order not to advance into the cranial space and not to damage the dura tissue 70d.

Releasing of the driving mechanism is further facilitated by the magnets 54a, 54b which are arranged in the cavities 50, 52 of the drive shaft 12 and of the drill head 14 respectively. The repelling force between the magnets 54a, 54b also forces the drill head 14 in its released state depicted in Fig. 5b. It is noted, that by applying magnets 54a, 54b of suitably chosen remanence the release of the drive mechanism would occur even if the proximal edges 44 of the grooves 46 were parallel with the rotational axis t, and even if the edges 44 were to run helically in the direction opposite the rotational direction. Accordingly, it is not necessary to provide helical grooves 46 that help transfer the relative rotation of the drill head 14 and the chipping head 16 around the rotational axis t into relative displacement along the rotational axis t. For example such embodiments are also conceivable wherein the grooves 46 are parallel with the rotational axis, thus only relative displacement in the direction of the rotational axis t is allowed between the drill head 14 and the chipping head 16 and no relative rotation is allowed. However, preferably the two solutions are applied in combination in order to increase security, i.e. the groove 46 is formed with a helical edge 44 running in the rotational direction, and magnets 54a, 54b are provided between the drive shaft 12 and the drill head 14 with the same magnetic poles facing each other.

The perforator assembly 10 according to the invention has for advantage that the drive mechanism realised through the first and second connecting profiles 20, 24 is independent from the release mechanism, which is realised by the repelling magnets 54a, 54b disposed in the cavities 50, 52 and optionally by the above described grooves 46 and bolt pins 48. Due to the independent release mechanism the drill head 14 does not disconnect from the drive shaft 12 as long as the distal displacement of the drill head 14 does not exceed the connection length 36. Consequently, if advancing of the perforator assembly 10 should be stayed for any reason during the drilling process or if the counter force acting on the drill head 14 temporarily decreases due to a change in the tissue quality, this does not necessarily result in decoupling of the drive mechanism. This also solves the problem of drilling through the thin bone plate 70c internally covering the bone tissue 70 of the crane, since by appropriate choice of the connection length 36, the drill head 14 only just perforates the internal bone plate 70c creating a disk 70c' from its material in front of the drill head 14 before coming to a stop.

A further advantage of the perforator assembly 10 according to the invention is that if the drive mechanism is disengaged - for example because the user is forced to temporarily pull back the perforator assembly 10 for any reason - then if the drill head 14 is pressed against the bone tissue 70 again then the wedge surfaces 34 on the second connecting profile 24 automatically direct the projections 30 of the first connection profile 20 into the clearances 35, i.e. into the position wherein the abutting surfaces 26 abut the driving surfaces 28. This greatly facilitates re-coupling of the drive mechanism.

The perforator assembly 10 according to the invention has for further advantage that the profile of the repelling force between the magnets 54a, 54b has been found to ensure surprisingly more satisfying drilling properties, than the application of a biasing spring arranged between the drive shaft 12 and the drill head 14. The magnetic repelling force falls off inversely with the square of the distance from the magnet's centre, whereas the spring force has linear distance dependence. Consequently, the magnetic repelling force ensures a more smooth operation and facilitates re-coupling of the drive mechanism in case of inadvertent disengagement. It is also more easy to dimension the perforator assembly 10 according to the present invention such that it only just cuts through the internal bone plate 70c before the drive mechanism is disconnected because the exerted magnetic force is high at the start of the disconnection process but it dies away fast in order to allow the drill head 12 to slow down while cutting through the internal bone plate 70c. In the prior art these properties have been ensured with the application of a second security spring, however, the non-linear force profile of the magnets 54a, 54b ensures superior operation as compared to counter acting springs with linear force profiles.

In addition, the release mechanism formed by the magnets 54a, 54b provides for a more convenient structure as compared to spring biasing means applied in the prior art: the perforator assembly is more simple to assemble and disassemble and to clean since the magnets 54a, 54b are retained in sealed off cavities 50, 52.

Various modifications to the above disclosed embodiments will be apparent to a person skilled in the art without departing from the scope of protection determined by the attached claims.

**Claims**

1. Perforator assembly for drilling bone tissue, comprising a drive shaft (12) having a rotation axis (t), a chipping head (16) having the same rotation axis (t), a drill head (14) arranged coaxially inside the chipping head (16); a first connecting profile (20) is provided at a proximal end (18) of the drill head (14), and a second connecting profile (24) is provided at a distal end (22) of the drive shaft (12), and the drill head (14) is arranged so as to be displaceable with respect to the chipping head (16) along the rotation axis (t) between a proximal position and a distal position, and in the proximal position the second connecting profile (24) at the distal end (22) of the drive shaft (12) cooperates with the first connecting profile (20) such as to transmit rotational motion from the drive shaft (12) to the drill head (14), and in the distal position the first connecting profile (20) at the proximal end (18) of the drill head (14) and the second connecting profile (24) on the distal end (22) of the drive shaft (12) are disengaged **characterised by** that a first magnet (54a) is arranged in a closed off cavity (50) at the proximal end (18) of the drill head (14), and a second magnet (54b) is arranged in a closed off cavity (52) at a distal end (22) of the drive shaft (12) such that same magnetic poles are facing each other.

2. The perforator assembly according to claim 1, **characterised by** that the magnets (54a, 54b) are made of neodymium.

3. The perforator assembly according to claims 1 or 2, **characterised by** that the magnets (54a, 54b) have a remanence of 1200 to 1300 mT.

4. The perforator assembly according to any one of claims 1 to 3, **characterised by** that the magnets (54a, 54b) are hermetically sealed off by closing plates (51, 53) respectively.

5. The perforator assembly according to any one of claims 1 to 4, **characterised by** that a connection is provided between the drill head (14) and the chipping head (16) for transforming relative rotation of the drill head (14) and the chipping head (16) around the rotational axis (t) into relative displacement along the rotational axis (t).

6. The perforator assembly according to claim 5, **characterised by** that the drill head (14) comprises a bore hole (40) through an external surface (38) of the drill head (14), and a groove (46) with a proximal helical edge (44) is provided in a wall (42) of the chipping head (16), and the connection for transforming relative rotation of the drill head (14) and the chipping head (16) around the rotational axis (t) into relative displacement is ensured by a bolt pin (48) arranged in the bore hole (40) of the drill head (14) and extending into the groove (46) of the chipping head (16), which bolt pin (48) is guided by the proximal helical edge (44).

7. The perforator assembly according to claim 6, **characterised by** that the bolt pin (48) is permanently fixed in the bore hole (40) of the drill head (14), and the groove (46) opens in the direction of the proximal end of the chipping head (16) through an opening (46a), and the opening (46a) is dimensioned such that the bolt pin (48) can pass through the opening (46a) when the drill head (14) is inserted into the chipping head (16).

8. The perforator assembly according to claim 6 or 7, **characterised by** that at least two, preferably three grooves (46) and corresponding number of bolt pins (48) are provided.

9. The perforator assembly according to any one of claims 1 to 10, **characterised by** that the first connecting profile (20) comprises at least one abutting surface (26), and the second connecting profile (24) comprises at least one driving surface (28), and preferably the at least one abutting surface (26) and the at least one driving surface (28) are rectangular.

10. The perforator assembly according to claim 9, **characterised by** that the first connecting profile (20) comprises at least two, preferably three abutting surfaces (26) that are formed rotation-symmetrically with respect to the rotational axis (t), each abutting surfaces (26) delimiting a projection (30) projecting parallel with the rotational axis (t), and the second connecting profile (24) comprises corresponding number of driving surfaces (28) that are formed rotation-symmetrically with respect to the rotational axis (t), each driving surface (28) being formed as a wall of a recess (32) parallel with the rotational axis (t), which recesses (32) are formed with a wedge surface (34) on their side opposite the driving surface (28), and the recesses (32) are dimensioned for receiving the projections (30) of the first connecting profile (20).

11. The perforator assembly according to any one of claims 1 to 10, **characterised by** that the drive shaft (12) and the chipping head (16) are surrounded and interconnected by a cylindrical casing (55) having a proximal portion (56) and a distal portion (57), the proximal portion (56) and the distal portion (57) being respectively provided with cooperating threads (56a, 56b), the distal portion (57) is permanently attached to the chipping head (17), and the proximal portion (56) is provided with a proximal flange (56b) for abutting a collar (58) provided around the drive shaft (12).

12. The perforator assembly according to claim 1, **characterised by** that the perforator assembly is a four-piece perforator assembly comprising:

- a first piece being the drive shaft (12) with rotation axis (t),
- a second piece being the chipping head (16) for arranging with the same rotation axis (t) and being permanently provided with a distal casing portion (57) at its proximal end,
- a third piece being the drill head (14) for coaxially inserting into the chipping head (16), the drill head (14) being provided with a bolt pin (48) extending from an outer surface of a side wall (38) of the drill head (14), and

- a fourth piece being a proximal casing portion (55) for holding the pieces together,

the magnets (54a, 54b) provided in the cavities (50, 52) are permanently sealed off by closing plates (51, 53) provided at the proximal end (18) of the drill head (14) and the distal end (22) of the drive shaft (12) respectively, a side wall (42) of the chipping head (16) is provided with a groove (46) the groove (46) opening in the direction of the proximal end of the chipping head (16) through an opening (46a), and the opening (46a) being dimensioned such that the bolt pin (48) can pass through the opening (46a) when the drill head (14) is inserted into the chipping head (16), the groove (46) allowing the bolt pin (48) to be displaceable with respect to the chipping head (16) between a first position corresponding to the proximal position of the drill head (14) and a second position corresponding to the distal position of the drill head (14), the proximal casing portion (56) and the distal casing portion (57) being respectively provided with cooperating threads (56a, 56b), and the proximal casing portion (56) is provided with a proximal flange (56b) for abutting a collar (58) provided around the drive shaft (12) when connected to the distal casing portion (57) via the threads (56a, 56b).

13. The perforator assembly according to claim 12, **characterised by** that the magnets (54a, 54b) are made of neodymium.

14. The perforator assembly according to claims 12 or 13, **characterised by** that the magnets (54a, 54b) have a remanence of 1200 to 1300 mT.

15. The perforator assembly according to any one of claims 12 to 14, **characterised by** that at least two, preferably three grooves (46) and corresponding number of bolt pins (48) are provided.

**Patentansprüche**

1. Perforatoranordnung zum Bohren von Knochengewebe, umfassend eine Antriebswelle (12), die eine Rotationsachse (t) aufweist, einen Zerspanungskopf (16), der dieselbe Rotationsachse (t) aufweist, einen Bohrkopf (14), der koaxial innerhalb des Zerspanungskopfs (16) angeordnet ist; wobei ein erstes Verbindungsprofil (20) an einem proximalen Ende (18) des Bohrkopfs (14) bereitgestellt ist und ein zweites Verbindungsprofil (24) an einem distalen Ende (22) der Antriebswelle (12) bereitgestellt ist und der Bohrkopf (14) derart angeordnet ist, um in Bezug auf den Zerspanungskopf (16) entlang der Rotationsachse (t) zwischen einer proximalen Position und einer distalen Position verlagerbar zu sein, und wobei in der proximalen Position das zweite Verbindungsprofil (24) an dem distalen Ende (22) der Antriebswelle (12) mit dem ersten Verbindungsprofil (20) zusammenwirkt, um Rotationsbewegung von der Antriebswelle (12) an den Bohrkopf (14) zu übertragen, und in der distalen Position das erste Verbindungsprofil (20) an dem proximalen Ende (18) des Bohrkopfs (14) und das zweite Verbindungsprofil (24) an dem distalen Ende (22) der Antriebswelle (12) außer Eingriff stehen, **dadurch gekennzeichnet, dass** ein erster Magnet (54a) in einem abgeschlossenen Hohlraum (50) an dem proximalen Ende (18) des Bohrkopfs (14) angeordnet ist und ein zweiter Magnet (54b) in einem abgeschlossenen Hohlraum (52) an einem distalen Ende (22) der Antriebswelle (12) angeordnet ist, sodass gleiche magnetische Pole einander zugewandt sind.

2. Perforatoranordnung nach Anspruch 1, **dadurch gekennzeichnet, dass** die Magneten (54a, 54b) aus Neodym hergestellt sind.

3. Perforatoranordnung nach Anspruch 1 oder 2, **dadurch gekennzeichnet, dass** die Magneten (54a, 54b) eine Remanenz von 1200 bis 1300 mT aufweisen.

4. Perforatoranordnung nach einem der Ansprüche 1 bis 3, **dadurch gekennzeichnet, dass** die Magneten (54a, 54b) jeweils durch Verschlussplatten (51, 53) hermetisch abgedichtet sind.

5. Perforatoranordnung nach einem der Ansprüche 1 bis 4, **dadurch gekennzeichnet, dass** eine Verbindung zwischen dem Bohrkopf (14) und dem Zerspanungskopf (16) zum Umwandeln relativer Rotation des Bohrkopfs (14) und des Zerspanungskopfs (16) um die Rotationsachse (t) herum in relative Verlagerung entlang der Rotationsachse (t) bereitgestellt ist.

6. Perforatoranordnung nach Anspruch 5, **dadurch gekennzeichnet, dass** der Bohrkopf (14) ein Bohrungsloch (40) durch eine externe Oberfläche (38) des Bohrkopfs (14) hindurch umfasst und eine Nut (46) mit einer proximalen spiralförmigen Kante (44) in einer Wand (42) des Zerspanungskopfs (16) bereitgestellt ist und die Verbindung zum Umwandeln von relativer Rotation des Bohrkopfs (14) und des Zerspanungskopfs (16) um die Rotationsachse (t) herum in relative Verlagerung durch einen Bolzenzapfen (48) sichergestellt ist, der in dem Bohrungsloch (40) des Bohrkopfs (14) angeordnet ist und sich in die Nut (46) des Zerspanungskopfs (16) hinein erstreckt, wobei der Bolzenzapfen (48) durch die proximale spiralförmige Kante (44) geführt wird.

7. Perforatoranordnung nach Anspruch 6, **dadurch gekennzeichnet, dass** der Bolzenzapfen (48) permanent in dem Bohrungsloch (40) des Bohrkopfs (14) fixiert ist und sich die Nut (46) in die Richtung des proximalen Endes des Zerspanungskopfs (16) durch eine Öffnung (46a) hindurch öffnet und die Öffnung (46a) derart dimensioniert ist, dass der Bolzenzapfen (48) durch die Öffnung (46a) hindurch verlaufen kann, wenn der Bohrkopf (14) in den Zerspanungskopf (16) hinein eingesetzt ist.

8. Perforatoranordnung nach Anspruch 6 oder 7, **dadurch gekennzeichnet, dass** mindestens zwei, vorzugsweise drei Nuten (46) und eine entsprechende Zahl von Bolzenzapfen (48) bereitgestellt sind.

9. Perforatoranordnung nach einem der Ansprüche 1 bis 10, **dadurch gekennzeichnet, dass** das erste Verbindungsprofil (20) mindestens eine Anstoßoberfläche (26) umfasst und das zweite Verbindungsprofil (24) mindestens eine Antriebsoberfläche (28) umfasst und vorzugsweise die mindestens eine Anstoßoberfläche (26) und die mindestens eine Antriebsoberfläche (28) rechteckig sind.

10. Perforatoranordnung nach Anspruch 9, **dadurch gekennzeichnet, dass** das erste Verbindungsprofil (20) mindestens zwei, vorzugsweise drei Anstoßoberflächen (26) umfasst, die in Bezug auf die Rotationsachse (t) rotationssymmetrisch gebildet sind, wobei jede der Anstoßoberflächen (26) einen Vorsprung (30) begrenzt, der parallel zu der Rotationsachse (t) hervorsteht, und das zweite Verbindungsprofil (24) eine entsprechende Zahl von Antriebsoberflächen (28) umfasst, die in Bezug auf die Rotationsachse (t) rotationssymmetrisch gebildet sind, wobei jede Antriebsoberfläche (28) als eine Wand einer Aussparung (32) parallel zu der Rotationsachse (t) gebildet ist, wobei die Aussparungen (32) mit einer Keiloberfläche (34) auf ihrer Seite gegenüber der Antriebsoberfläche (28) gebildet sind und die Aussparungen (32) zum Empfangen der Vorsprünge (30) des ersten Verbindungsprofils (20) dimensioniert sind.

11. Perforatoranordnung nach einem der Ansprüche 1 bis 10, **dadurch gekennzeichnet, dass** die Antriebswelle (12) und der Zerspanungskopf (16) durch ein zylindrisches Gehäuse (55) umgeben und miteinander verbunden sind, das einen proximalen Abschnitt (56) und einen distalen Abschnitt (57) aufweist, wobei der proximale Abschnitt (56) und der distale Abschnitt (57) jeweils mit zusammenwirkenden Gewinden (56a, 56b) bereitgestellt sind, wobei der distale Abschnitt (57) permanent an dem Zerspanungskopf (17) angebracht ist und der proximale Abschnitt (56) mit einem proximalen Flansch (56b) zum Anstoßen an einen Kragen (58), der um die Antriebswelle (12) herum bereitgestellt ist, bereitgestellt ist.

**12.** Perforatoranordnung nach Anspruch 1, **dadurch gekennzeichnet, dass** die Perforatoranordnung eine vierteilige Perforatoranordnung ist, umfassend:

- ein erstes Teil, das die Antriebswelle (12) mit Rotationsachse (t) ist,
- ein zweites Teil, das der Zerspanungskopf (16) zum Anordnen mit derselben Rotationsachse (t) ist und permanent mit einem distalen Gehäuseabschnitt (57) an seinem proximalen Ende bereitgestellt ist,
- ein drittes Teil, das der Bohrkopf (14) ist, zum koaxialen Einsetzen in den Zerspanungskopf (16) hinein, wobei der Bohrkopf (14) mit einem Bolzenzapfen (48) bereitgestellt ist, der sich von einer äußeren Oberfläche einer Seitenwand (38) des Bohrkopfs (14) erstreckt, und
- ein viertes Teil, das ein proximaler Gehäuseabschnitt (55) zum Zusammenhalten der Teile ist,

wobei die Magneten (54a, 54b), die in den Hohlräumen (50, 52) bereitgestellt sind, permanent durch Verschlussplatten (51, 53) abgedichtet sind, die jeweils an dem proximalen Ende (18) des Bohrkopfs (14) und an dem distalen Ende (22) der Antriebswelle (12) bereitgestellt sind, wobei eine Seitenwand (42) des Zerspanungskopfs (16) mit einer Nut (46) bereitgestellt ist, wobei sich die Nut (46) in die Richtung des proximalen Endes des Zerspanungskopfs (16) durch eine Öffnung (46a) hindurch öffnet und die Öffnung (46a) derart dimensioniert ist, dass der Bolzenzapfen (48) durch die Öffnung (46a) hindurch verlaufen kann, wenn der Bohrkopf (14) in den Zerspanungskopf (16) hinein eingesetzt ist, wobei die Nut (46) dem Bolzenzapfen (48) erlaubt, in Bezug auf den Zerspanungskopf (16) zwischen einer ersten Position, die der proximalen Position des Bohrkopfs (14) entspricht, und einer zweiten Position, die der distalen Position des Bohrkopfs (14) entspricht, verlagerbar zu sein, wobei der proximale Gehäuseabschnitt (56) und der distale Gehäuseabschnitt (57) jeweils mit zusammenwirkenden Gewinden (56a, 56b) bereitgestellt sind und der proximale Gehäuseabschnitt (56) mit einem proximalen Flansch (56b) zum Anstoßen an einen Kragen (58), der um die Antriebswelle (12) herum bereitgestellt ist, bereitgestellt ist, wenn er mit dem distalen Gehäuseabschnitt (57) über die Gewinde (56a, 56b) verbunden ist.

**13.** Perforatoranordnung nach Anspruch 12, **dadurch gekennzeichnet, dass** die Magneten (54a, 54b) aus Neodym hergestellt sind.

**14.** Perforatoranordnung nach Anspruch 12 oder 13, **dadurch gekennzeichnet, dass** die Magneten (54a, 54b) eine Remanenz von 1200 bis 1300 mT aufweisen.

**15.** Perforatoranordnung nach einem der Ansprüche 12 bis 14, **dadurch gekennzeichnet, dass** mindestens zwei, vorzugsweise drei Nuten (46) und eine entsprechende Zahl von Bolzenzapfen (48) bereitgestellt sind.

## Revendications

**1.** Ensemble perforateur pour percer un tissu osseux, comprenant un arbre d'entraînement (12) ayant un axe de rotation (t), une tête déchiqueteuse (16) ayant le même axe de rotation (t), une tête de perçage (14) agencée de manière coaxiale à l'intérieur de la tête déchiqueteuse (16) ; un premier profil de connexion (20) est fourni à une extrémité proximale (18) de la tête de perçage (14), et un second profil de connexion (24) est fourni à une extrémité distale (22) de l'arbre d'entraînement (12), et la tête de perçage (14) est agencée afin de pouvoir être déplacée par rapport à la tête déchiqueteuse (16) le long de l'axe de rotation (t) entre une position proximale et une position distale, et dans la position proximale le second profil de connexion (24) à l'extrémité distale (22) de l'arbre d'entraînement (12) coopère avec le premier profil de connexion (20) de façon à transmettre un mouvement de rotation provenant de l'arbre d'entraînement (12) à la tête de perçage (14), et dans la position distale le premier profil de connexion (20) à l'extrémité proximale (18) de la tête de perçage (14) et le second profil de connexion (24) sur l'extrémité distale (22) de l'arbre d'entraînement (12) sont désaccouplés **caractérisé en ce qu'**un premier aimant (54a) est agencé dans une cavité fermée (50) à l'extrémité proximale (18) de la tête de perçage (14), et un second aimant (54b) est agencé dans une cavité fermée (52) à une extrémité distale (22) de l'arbre d'entraînement (12) d'une manière telle que les mêmes pôles magnétiques sont l'un en face de l'autre.

**2.** Ensemble perforateur selon la revendication 1, **caractérisé en ce que** les aimants (54a, 54b) sont faits de néodyme.

**3.** Ensemble perforateur selon les revendications 1 ou 2, **caractérisé en ce que** les aimants (54a, 54b) ont une rémanence de 1200 à 1300 mT.

**4.** Ensemble perforateur selon l'une quelconque des revendications 1 à 3, **caractérisé en ce que** les aimants (54a, 54b) sont hermétiquement scellés par des plaques de fermeture (51, 53) respectivement.

**5.** Ensemble perforateur selon l'une quelconque des revendications 1 à 4, **caractérisé en ce qu'**une connexion est fournie entre la tête de perçage (14) et la tête déchiqueteuse (16) pour transformer une rotation relative de la tête de perçage (14) et de la tête déchiqueteuse (16) autour de l'axe de rotation (t) en déplacement relatif le long de l'axe de rotation (t).

6. Ensemble perforateur selon la revendication 5, **caractérisé en ce que** la tête de perçage (14) comprend un trou de forage (40) à travers une surface externe (38) de la tête de perçage (14), et une rainure (46) ayant un bord hélicoïdal proximal (44) est fournie dans une paroi (42) de la tête déchiqueteuse (16), et la connexion pour transformer une rotation relative de la tête de perçage (14) et de la tête déchiqueteuse (16) autour de l'axe de rotation (t) en déplacement relatif est assurée par une tige de boulon (48) agencée dans le trou de forage (40) de la tête de perçage (14) et s'étendant dans la rainure (46) de la tête déchiqueteuse (16), laquelle tige de boulon (48) est guidée par le bord hélicoïdal proximal (44).

7. Ensemble perforateur selon la revendication 6, **caractérisé en ce que** la tige de boulon (48) est fixée en permanence dans le trou de forage (40) de la tête de perçage (14), et la rainure (46) s'ouvre dans la direction de l'extrémité proximale de la tête déchiqueteuse (16) à travers une ouverture (46a), et l'ouverture (46a) est dimensionnée de façon à ce que la tige de boulon (48) puisse passer à travers l'ouverture (46a) quand la tête de perçage (14) est insérée dans la tête déchiqueteuse (16).

8. Ensemble perforateur selon la revendication 6 ou 7, **caractérisé en ce qu'**au moins deux, de préférence trois rainures (46) et un nombre correspondant de tiges de boulon (48) sont fournis.

9. Ensemble perforateur selon l'une quelconque des revendications 1 à 10, **caractérisé en ce que** le premier profil de connexion (20) comprend au moins une surface de butée (26), et le second profil de connexion (24) comprend au moins une surface d'entraînement (28), et de préférence l'au moins une surface de butée (26) et l'au moins une surface d'entraînement (28) sont rectangulaires.

10. Ensemble perforateur selon la revendication 9, **caractérisé en ce que** le premier profil de connexion (20) comprend au moins deux, de préférence trois surfaces de butée (26) qui sont formées de manière symétrique en rotation par rapport à l'axe de rotation (t), chaque surface de butée (26) délimitant une saillie (30) faisant saillie parallèlement à l'axe de rotation (t), et le second profil de connexion (24) comprend un nombre correspondant de surfaces d'entraînement (28) qui sont formées de manière symétrique en rotation par rapport à l'axe de rotation (t), chaque surface d'entraînement (28) étant formée sous la forme d'une paroi d'un évidement (32) parallèle à l'axe de rotation (t), lesquels évidements (32) sont formés avec une surface en coin (34) sur leur côté opposé à la surface d'entraînement (28), et les évidements (32) sont dimensionnés pour recevoir les saillies (30) du premier profil de connexion (20).

11. Ensemble perforateur selon l'une quelconque des revendications 1 à 10, **caractérisé en ce que** l'arbre d'entraînement (12) et la tête déchiqueteuse (16) sont entourés et interconnectés par un carter cylindrique (55) ayant une partie proximale (56) et une partie distale (57), la partie proximale (56) et la partie distale (57) étant respectivement pourvues de filetages qui coopèrent (56a, 56b), la partie distale (57) est fixée en permanence à la tête déchiqueteuse (17), et la partie proximale (56) est pourvue d'un rebord proximal (56b) pour venir en butée contre un collier (58) fourni autour de l'arbre d'entraînement (12).

12. Ensemble perforateur selon la revendication 1, **caractérisé en ce que** l'ensemble perforateur est un ensemble perforateur à quatre pièces comprenant :

   - une première pièce étant l'arbre d'entraînement (12) avec un axe de rotation (t),
   - une deuxième pièce étant la tête déchiqueteuse (16) pour un agencement avec le même axe de rotation (t) et étant pourvue en permanence d'une partie distale de carter (57) à son extrémité proximale,
   - une troisième pièce étant la tête de perçage (14) pour une insertion coaxiale dans la tête déchiqueteuse (16), la tête de perçage (14) étant pourvue d'une tige de boulon (48) s'étendant à partir d'une surface externe d'une paroi latérale (38) de la tête de perçage (14), et
   - une quatrième pièce étant une partie proximale de carter (55) pour maintenir les pièces ensemble,

les aimants (54a, 54b) fournis dans les cavités (50, 52) sont scellés en permanence par des plaques de fermeture (51, 53) fournies à l'extrémité proximale (18) de la tête de perçage (14) et l'extrémité distale (22) de l'arbre d'entraînement (12) respectivement, une paroi latérale (42) de la tête déchiqueteuse (16) est pourvue d'une rainure (46) la rainure (46) s'ouvrant dans la direction de l'extrémité proximale de la tête déchiqueteuse (16) à travers une ouverture (46a), et l'ouverture (46a) étant dimensionnée de façon à ce que la tige de boulon (48) puisse passer à travers l'ouverture (46a) quand la tête de perçage (14) est insérée dans la tête déchiqueteuse (16), la rainure (46) permettant à la tige de boulon (48) de pouvoir être déplacée par rapport à la tête déchiqueteuse (16) entre une première position correspondant à la position proximale de la tête de perçage (14) et une seconde position correspondant à la position distale de la tête de perçage (14), la partie proximale de carter (56) et la partie distale de carter (57) étant respectivement pourvues de filetages qui coopèrent (56a, 56b), et la partie proximale de carter (56) est pourvue d'un rebord proximal (56b) pour venir en butée contre un collier (58) fourni autour de l'arbre d'entraînement (12) quand elle est connectée à la partie distale de carter (57) via les filetages (56a, 56b).

13. Ensemble perforateur selon la revendication 12, **caractérisé en ce que** les aimants (54a, 54b) sont faits néodyme.

14. Ensemble perforateur selon les revendications 12 ou 13, **caractérisé en ce que** les aimants (54a, 54b) ont une rémanence de 1200 à 1300 mT.

15. Ensemble perforateur selon l'une quelconque des revendications 12 à 14, **caractérisé en ce qu'**au moins deux, de préférence trois rainure (46) et un nombre correspondant de tiges de boulon (48) sont fournis.

Fig. 1

Fig.1b

Fig. 2a

Fig. 2b

Fig. 2c

Fig. 3a

Fig. 3b

Fig. 4a

Fig. 4b

Fig. 5a

Fig. 5b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015150844 A